# FASCICULE DE BREVET EUROPEEN

(11) **EP 1 526 861 B1**
(45) Date de publication et mention de la délivrance du brevet: **21.11.2007**
(21) Numéro de dépôt: 03750850.4
(22) Date de dépôt: 28.07.2003
(51) Int. Cl.: A61K 36/54, C11B 1/00, C07D 307/00

(54) **PROCEDE D'OBTENTION D'UN EXTRAIT DE FEUILLES D'AVOCATIER RICHE EN LIPIDES FURANIQUES**
VERFAHREN ZUR GEWINNUNG EINES AVOCADOBLATTEXTRAKTES, DER REICH AN FURANLIPIDE IST
METHOD FOR PRODUCING AVOCADO LEAVES EXTRACT RICH IN FURAN LIPIDS

(30) Priorité: 30.07.2002 FR 0209666; 30.07.2002 US 206995
(43) Date de publication de la demande: 04.05.2005
(73) Titulaire: Laboratoires Expanscience, 92400 Courbevoie (FR)
(72) Inventeur: PICCIRILLI, Antoine, F-78000 Versailles (FR); LEGRAND, Jacques, F-61290 Neuilly sur Eure (FR)
(74) Mandataire: Le Forestier, Eric
(86) Numéro de dépôt international: PCT/FR2003/002377
(87) Numéro de publication internationale: WO 2004/012752

(56) Documents cités:
- WO-A-01/21605
- US-A- 5 262 163
- FARINES M ET AL: "Influence of Avocado Oil Processing on the Nature of Some Unsaponifiable Constituents" JOURNAL OF THE AMERICAN OIL CHEMISTS' SOCIETY, AMERICAN OIL CHEMISTS' SOCIETY. CHAMPAIGN, US, vol. 72, no. 4, 1995, pages 473-476, XP002144061 ISSN: 0003-021X
- PICCARDI N. ET AL.: "Specific furans from avocado : a new tool in dermatology." J. INVEST. DERMATOL., vol. 117, no. 2, août 2001 (2001-08), page 398 XP009007184

## Description

La présente invention concerne un procédé d'obtention d'un extrait végétal de feuilles d'avocatier riche en lipides furaniques.

L'avocat comprend de manière connue des lipides particuliers de type furanique, dont le principal composant est un furane linoléique :

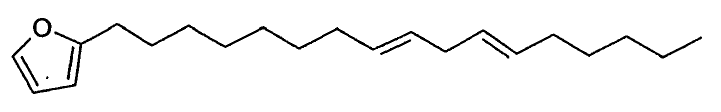

Ainsi, « Par lipides furaniques d'avocat », on entend selon l'invention les composants répondant à la formule : 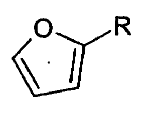 dans laquelle R est une chaîne linéaire hydrocarbonée en C₁₁-C₁₉ de préférence C₁₃-C₁₇ saturée ou comprenant une ou plusieurs insaturations éthyléniques ou acétyléniques.

Ces lipides furaniques d'avocat ont été décrits notamment dans Farines, M. et al, 1995, J. of Am. Oil Chem. Soc. 72, 473.

Actuellement, les voies de synthèse connues d'obtention de lipides furaniques et couramment utilisées partent des avocats sous forme de fruits comme matière première.

Compte tenu de l'intérêt thérapeutique des lipides furaniques d'avocat pour leur action bénéfique et curative sur le tissu conjonctif, notamment dans les pathologies inflammatoires telles que l'arthrose, les parodontites et la sclérodermie, et de leur coût élevé en général, il existe donc un intérêt fort à trouver des voies alternatives de préparation de ces lipides furaniques d'avocat.

La demanderesse a ainsi mis au point un procédé, permettant d'obtenir un extrait végétal de feuille d'avocatier riche en lipides furaniques, à savoir d'une teneur variant de 20 à 80% et de préférence 30 à 50%.

Ce procédé, dont la matière première est constituée de feuilles d'avocatier, comprend les étapes suivantes :
- une étape d'extraction liquide-solide suivie d'une évaporation sous vide et,
- une étape de traitement thermique entre 80 et 120°C.

Ces deux étapes peuvent être mises en oeuvre dans cet ordre ou dans l'ordre inverse.

Les feuilles d'avocatier peuvent provenir des avocatiers appartenant aux variétés suivantes : *Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Red* ou *Alpha Krome* et plus avantageusement aux variétés *Hass, Fuerte* et *Reed.*

Plusieurs solvants peuvent être utilisés lors de l'étape d'extraction liquide-solide des feuilles, ayant éventuellement préalablement subi une déshydratation. Les solvants peuvent être choisis dans le groupe constitué par les alcanes, les alcanes halogénés, les éthers, les esters, les alcools, les composés aromatiques et les fluides supercritiques. On préfère, seul ou en mélange, l'hexane, l'éthanol, le méthanol, le chloroforme, le dichlorométhane, et l'acétate d'éthyle.

L'étape de traitement thermique, qu'elle intervienne directement sur les feuilles ou au stade de l'extrait végétal, est mise en oeuvre à des températures comprises entre 80 et 120°C. Ce traitement thermique peut durer de 5 à 72 heures.

Selon le procédé de l'invention, l'étape de traitement thermique peut se faire en présence ou non d'un catalyseur acide.

On entend par catalyseurs acides au sens large les catalyseurs minéraux et organiques dits homogènes tels que les acides chlorhydrique, sulfurique, acétique ou paratoluènesulfonique mais aussi, et de préférence, les catalyseurs solides hétérogènes tel's que la silice; l'alumine, les silices-alumines, les zircones, les zéolithes, les résines acides. On choisira en particulier les alumines acides de grandes surfaces spécifiques, c'est à dire au moins égales à 200 m²/g.

Le procédé peut également comprendre une étape, préalable à l'extraction liquide-solide, de déshydratation des feuilles d'avocatier.

On entend plus généralement par déshydratation des feuilles d'avocatier, l'ensemble des techniques connues de l'homme de métier et qui permettent d'extraire l'eau d'un composé. Parmi ces techniques on peut citer le séchage sous courant d'air chaud ou sous atmosphère contrôlée (ex. azote), à pression atmosphérique ou sous vide, en couche épaisse ou couche mince, mais encore le séchage par micro-ondes, le séchage par pulvérisation, la lyophilisation et la déshydratation osmotique en solution (osmose directe) ou en phase solide (ex. séchage en sacs osmotiques).

D'une façon générale, la température lors de cette étape de déshydratation sera de préférence maintenue, quelle que soit la technique employée, inférieure ou égale à 80°C.

Par ailleurs, on préfère dans le cadre de la présente invention arrêter la déshydratation lorsque l'humidité résiduelle atteint le seuil de 5%.

Dans le cadre du présent procédé, pour des raisons de facilités de mise en oeuvre industrielle et pour des raisons de coût, le séchage en séchoirs ventilés, en couche mince et sous courant d'air chaud, à une température comprise entre 70 et 75°C est préféré. La durée de l'opération peut varier de 5 à 72 heures.

Selon une première variante, le procédé est mis en oeuvre selon la succession d'étapes suivantes :
- Etape 1 _{:} traitement thermique des feuilles d'avocatier,
- Etape 2 _{:} extraction liquide-solide des feuilles d'avocatier,
- Etape 3 _{:} évaporation sous vide du solvant jusqu'à obtention d'un extrait végétal désolvanté.

Selon une deuxième variante, le procédé est mis en oeuvre selon la succession d'étapes suivante
- Etape 1 : extraction liquide-solide des feuilles d'avocatier,
- Etape 2 _{:} évaporation sous vide du solvant jusqu'à obtention d'un extrait végétal désolvanté,
- Etape 3 _{:} traitement thermique de l'extrait végétal.

Selon une dernière variante, le procédé est mis en oeuvre selon la succession d'étapes suivante
- Etape 1 : déshydratation des feuilles d'avocatier,
- Etape 2 : extraction liquide-solide des feuilles d'avocatier,
- Etape 3 : évaporation sous vide du solvant jusqu'à obtention d'un extrait végétal,
- Etape 4 : traitement thermique de l'extrait végétal.

Les feuilles sont avantageusement broyées avant toute opération effectuée dans le cadre du procédé de l'invention.

Les exemples suivants, non limitatifs, illustrent l'invention.

**Exemple 1 :** un kilogramme de feuilles d'avocatier de la variété *Hass,* est soigneusement broyé. Le broyat obtenu (320 g) est mis en contact avec 2 litres d'éthanol puis porté à reflux pendant 5 heures. Une fois refroidi le mélange est filtré sur büchner et le gâteau lavé par trois fois 100 ml d'éthanol. Le solvant est ensuite évaporé sous vide à l'aide d'un évaporateur rotatif, puis tiré sous vide pendant encore une heure après élimination totale du solvant. L'extrait obtenu est alors mis à l'étuve à 100°C pendant 24 heures, puis analysé. On obtient selon ce procédé 23 g d'extrait.

L'analyse physico-chimique et chromatographique de cet extrait a donné les résultats suivants :
- Résidu à l'incinération : < 0,1 %
- Teneurs en composés volatils : < 0,1 %
- Teneur en lipides furaniques : 42 %

**Exemple 2 :** un kilogramme de feuilles d'avocatier de la variété Fuerte, est soigneusement broyé. Le broyat obtenu (305 g) est mis en contact avec 2 litres d'hexane puis porté à reflux pendant 5 heures. Une fois refroidi, le mélange est filtré sur büchner et le gâteau lavé par trois fois 100 ml d'hexane. Une fois rassemblées, les phases hexaniques sont séchées en présence de Na₂SO₄. L'hexane est ensuite évaporé sous vide à l'aide d'un évaporateur rotatif, puis tiré sous vide pendant encore une heure après élimination totale du solvant. L'extrait obtenu est alors mis à l'étuve à 100°C pendant 24 heures, puis analysé. On obtient selon ce procédé 26 g d'extrait.

L'analyse physico-chimique et chromatographique de cet extrait a donné les résultats suivants :
- Résidu à l'incinération : < 0,1 %
- Teneurs en composés volatils : < 0,1 %
- Teneur en lipides furaniques : 46 %

**Exemple 3 :** un kilogramme de feuilles d'avocatier de la variété *Hass,* est soigneusement broyé. Le broyat est mis en contact, sous agitation, pendant 5 heures, avec 10 litres de méthanol, 10 litres de chloroforme et 10 litres d'eau salée (à 1% de NaCl). Le mélange est ensuite centrifugé et le culot solide éliminé. La phase liquide inférieure est récupérée puis séchée sur Na₂SO₄. Les solvants sont alors évaporés sous vide à l'aide d'un évaporateur rotatif, puis tiré sous vide pendant encore une heure après élimination totale des solvants. L'extrait obtenu est alors mis à l'étuve à 100°C pendant 24 heures, puis analysé. On obtient selon ce procédé 21 g d'extrait.

L'analyse physico-chimique et chromatographique de cet extrait a donné les résultats suivants :
- Résidu à l'incinération : < 0,1 %
- Teneurs en composés volatils : < 0,1 %
- Teneur en lipides furaniques : 46 %

**Exemple 4 :** un kilogramme de feuilles d'avocatier de la variété *Hass,* est soigneusement broyé. Le broyat est ensuite séché à l'étuve, sous un courant d'air chaud, à 110°C pendant 24 heures. On récupère alors 302 g de matière sèche. Cette dernière est alors mise en contact avec 2 litres d'hexane puis porté à reflux pendant 5 heures. Une fois refroidi, le mélange est filtré sur büchner et le gâteau lavé par trois fois 100 ml d'hexane. L'hexane est ensuite évaporé sous vide à l'aide d'un évaporateur rotatif, puis tiré sous vide pendant encore une heure après élimination totale du solvant. On obtient selon ce procédé 31 g d'extrait.

L'analyse physico-chimique et chromatographique de cet extrait a donné les résultats suivants :
- Résidu à l'incinération : < 0,1 %
- Teneurs en composés volatils : < 0,1 %
- Teneur en lipides furaniques : 41 %

**Exemple 5 :** un kilogramme de feuilles d'avocatier de la variété *Hass,* est soigneusement broyé. Le broyat est ensuite séché à l'étuve, sous un courant d'air chaud, à 70°C pendant 72 heures. On récupère alors 315 g de matière sèche. L'humidité résiduelle présente dans les feuilles est alors de 4,8 %. Cette matière déshydratée est alors mise en contact avec 2 litres d'hexane puis portée à reflux pendant 5 heures. Une fois refroidi, le mélange est filtré sur büchner et le gâteau lavé par trois fois 100 ml d'hexane. L'hexane est ensuite évaporé sous vide à l'aide d'un évaporateur rotatif, puis tiré sous vide pendant encore une heure après élimination totale du solvant. L'extrait obtenu est alors mis à l'étuve à 100°C pendant 24 heures, puis analysé. On obtient alors selon ce procédé 23 g d'extrait.

L'analyse physico-chimique et chromatographique de cet extrait a donné les résultats suivants :
- Résidu à l'incinération : < 0,1 %
- Teneurs en composés volatils : < 0,1 %
- Teneur en lipides furaniques : 38 %

### Exemple comparatif / Procédé sans chauffage poussé des feuilles

**Exemple :** un kilogramme de feuilles d'avocatier de la variété *Hass,* est soigneusement broyé. Le broyat est ensuite séché à l'étuve; sous un courant d'air chaud, à 60°C pendant 96 heures. On récupère alors 307 g de matière sèche. Cette dernière est alors mise en contact avec 2 litres d'hexane puis porté à reflux pendant 5 heures. Une fois refroidi, le mélange est filtré sur büchner et le gâteau lavé par trois fois 100 ml d'hexane. L'hexane est ensuite évaporé sous vide à l'aide d'un évaporateur rotatif, à 60°C, puis tiré sous vide pendant encore une heure après élimination totale du solvant. On obtient selon ce procédé 27 g d'extrait.

L'analyse physico-chimique et chromatographique de cet extrait a donné les résultats suivants :
- Résidu à l'incinération : < 0,1 %
- Teneurs en composés volatils : < 0,1 %
- Teneur en lipides furaniques : 2 %

Conclusion : en absence de chauffage poussé des feuilles, de l'extrait obtenu ou de l'extrait obtenu des feuilles fraîches ou déshydratées après extraction à l'aide d'un solvant, l'extrait final obtenu ne contient pratiquement pas de lipides furaniques (teneur largement inférieure à 5 %).

## Revendications

1. Procédé d'obtention d'un extrait végétal ayant une teneur en lipides furaniques de 20 à 80%, **caractérisé en ce que** la matière première est constituée des feuilles d'avocatier et le procédé comprend les étapes suivantes :
- une étape d'extraction liquide-solide suivie d'une évaporation sous vide et
- une étape de traitement thermique entre 80 et 120°C,
ces deux étapes pouvant être mises en oeuvre dans cet ordre ou dans l'ordre inverse.

2. Procédé d'obtention d'un extrait végétal selon la revendication 1, **caractérisé en ce que** les feuilles d'avocatier proviennent des avocatiers appartenant aux variétés suivantes : *Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Red* ou *Alpha Krome.*

3. Procédé d'obtention d'un extrait végétal selon l'une quelconque des revendications 1 ou 2, **caractérisé en ce que** le traitement thermique est mis en oeuvre pendant une durée variant entre 5 et 72 heures.

4. Procédé d'obtention d'un extrait végétal selon l'une quelconque des revendications 1 à 3, **caractérisé en ce que** le ou les solvants appropriés lors de l'étape d'extraction liquide-solide sont choisis dans le groupe constitué par les alcanes, les alcanes halogénés, les éthers, les esters, les alcools, les composés aromatiques et les fluides supercritiques.

5. Procédé d'obtention d'un extrait végétal selon la revendication 4, **caractérisé en ce que** le ou les solvants appropriés lors de l'étape d'extraction liquide-solide sont choisis dans le groupe constitué par l'hexane, l'éthanol, le méthanol, le chloroforme, le dichlorométhane, et l'acétate d'éthyle, pris seul ou en mélange.

6. Procédé d'obtention d'un extrait végétal selon l'une quelconque des revendications 1 à 5, **caractérisé en ce que** le procédé comprend une étape préalable à l'extraction, de déshydratation des feuilles d'avocatier.

7. Procédé d'obtention d'un extrait végétal selon la revendication 6, **caractérisé en ce que** la déshydratation est choisie dans le groupe constitué par le séchage sous courant d'air chaud ou sous atmosphère contrôlée, la séchage à pression atmosphérique ou sous vide, en couche épaisse ou couche mince, le séchage par micro-ondes, le séchage par pulvérisation, la lyophilisation et la déshydratation osmotique en solution ou en phase solide.

8. Procédé d'obtention d'un extrait végétal selon la revendication 7, **caractérisé en ce que** la déshydratation consiste en un séchage en séchoirs ventilés, en couche mince et sous courant d'air chaud, à une température comprise entre 70 et 75°C.

9. Procédé d'obtention d'un extrait végétal selon l'une quelconque des revendications 1 à 8, **caractérisé en ce que** le procédé est mis en oeuvre selon la succession d'étapes suivantes :
- Etape 1 : traitement thermique des feuilles d'avocatier,
- Etape 2 : extraction liquide-solide des feuilles d'avocatier,
- Etape 3 : évaporation sous vide du solvant jusqu'à obtention d'un extrait végétal désolvanté.

10. Procédé d'obtention d'un extrait végétal selon l'une quelconque des revendications 1 à 8, **caractérisé en ce que** le procédé est mis en oeuvre selon la succession d'étapes suivantes :
- Etape 1 : extraction liquide-solide des feuilles d'avocatier,
- Etape 2 : évaporation sous vide du solvant jusqu'à obtention d'un extrait végétal désolvanté,
- Etape 3 : traitement thermique de l'extrait végétal.

11. Procédé d'obtention d'un extrait végétal selon l'une quelconque des revendications 1 à 8, **caractérisé en ce que** le procédé est mis en oeuvre selon la succession d'étapes suivantes :
- Etape 1 : déshydratation des feuilles d'avocatier,
- Etape 2 : extraction liquide-solide des feuilles d'avocatier,
- Etape 3 : évaporation sous vide du solvant jusqu'à obtention d'un extrait végétal,
- Etape 4 : traitement thermique de l'extrait végétal.

## Claims

1. Method for obtaining a plant extract having a furan lipid content of 20 to 80 %, **characterised in that** the raw material is constituted by avocado leaves, and the method includes the following steps:
- a step of liquid-solid extraction followed by vacuum evaporation, and,
- a heat treatment step between 80 and 120 °C,
which two steps can be implemented in this order or in the reverse order.

2. Method for obtaining a plant extract according to claim 1, **characterised in that** the avocado leaves come from avocados of the following varieties: Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Red or Alpha Krome.

3. Method for obtaining a plant extract according to either one of claims 1 or 2, **characterised in that** the heat treatment is implemented for a duration ranging from 5 to 72 hours.

4. Method for obtaining a plant extract according to any one of claims 1 to 3, **characterised in that** the suitable solvent(s) in the liquid-solid extraction step are chosen from the group consisting of alkanes, halogenated alkanes, ethers, esters, alcohols, aromatic compounds and supercritical fluids.

5. Method for obtaining a plant extract according to claim 4, **characterised in that** suitable solvent(s) in the liquid-solid extraction step are chosen from the group consisting of hexane, ethanol, methanol, chloroform, dichloromethane and ethyl acetate, taken alone or in a mixture.

6. Method for obtaining a plant extract according to any one of claims 1 to 5, **characterised in that** the method includes a step prior to the extraction, of dehydration of the avocado leaves.

7. Method for obtaining a plant extract according to claim 6, **characterised in that** the dehydration technique is chosen from the group consisting of drying under a hot air current or under a controlled atmosphere, drying at atmospheric pressure or under a vacuum, in a thick layer or a thin layer, microwave drying, spray drying, lyophilization and osmotic dehydration in solution or in the solid phase.

8. Method for obtaining a plant extract according to claim 7, **characterised in that** the dehydration consists of drying in ventilated driers, in a thin layer and under a hot air current, at a temperature between 70 and 75 °C.

9. Method for obtaining a plant extract according to any one of claims 1 to 8, **characterised in that** the method is implemented according to the following series of steps:
- step 1: heat treatment of avocado leaves,
- step 2: liquid-solid extraction of avocado leaves,
- step 3: vacuum evaporation of the solvent until a desolvented plant extract has been obtained.

10. Method for obtaining a plant extract according to any one of claims 1 to 8, **characterised in that** the method is implemented according to the following series of steps:
- step 1: liquid-solid extraction of avocado leaves,
- step 2: vacuum evaporation of the solvent until a desolvented plant extract has been obtained,
- step 3: heat treatment of the plant extract.

11. Method for obtaining a plant extract according to any one of claims 1 to 8, **characterised in that** the method is implemented according to the following series of steps:
- step 1: dehydration of the avocado leaves,
- step 2: liquid-solid extraction of avocado leaves,
- step 3: vacuum evaporation of the solvent until a desolvented plant extract has been obtained,
- step 4: heat treatment of the plant extract.

## Patentansprüche

1. Verfahren zum Erhalt eines Pflanzenextrakts mit einem Gehalt an furanischen Lipiden von 20 bis 80 %, **dadurch gekennzeichnet, dass** das Ausgangsmaterial aus Avocadobaum-Blättern besteht und das Verfahren die folgenden Schritte umfasst:
- einen Flüssig-Fest-Extraktionsschritt, gefolgt von einem Verdampfen unter Vakuum, und
- einen Wärmebehandlungsschritt zwischen 80 und 120°C,
wobei diese zwei Schritte in dieser Reihenfolge oder in umgekehrter Reihenfolge durchgeführt werden können.

2. Verfahren zum Erhalt eines Pflanzenextrakts nach Anspruch 1, **dadurch gekennzeichnet, dass** die Avocadobaum-Blätter von Avocadobäumen stammen, die den folgenden Varietäten angehören: *Hass, Fuerte, Ettinger, Bacon, Nabal, Anaheim, Lula, Reed, Zutano, Queen, Criola Selva, Mexicana Canta, Region Dschang, Hall, Booth, Peterson, Collinson Red* oder *Alpha Krome.*

3. Verfahren zum Erhalt eines Pflanzenextrakts nach irgendeinem der Ansprüche 1 oder 2, **dadurch gekennzeichnet, dass** die Wärmebehandlung über eine Dauer durchgeführt wird, die zwischen 5 und 72 Stunden variiert.

4. Verfahren zum Erhalt eines Pflanzenextrakts nach irgendeinem der Ansprüche 1 bis 3, **dadurch gekennzeichnet, dass** das oder die Lösungsmittel, die beim Schritt der Flüssig-Fest-Extraktion geeignet sind, ausgewählt sind aus der Gruppe bestehend aus Alkanen, halogenierten Alkanen, Ethern, Estern, Alkoholen, aromatischen Verbindungen und superkritischen Fluiden.

5. Verfahren zum Erhalt eines Pflanzenextrakts nach Anspruch 4, **dadurch gekennzeichnet, dass** das oder die Lösungsmittel, die beim Schritt der Fest-Flüssig-Extraktion geeignet sind, ausgewählt sind aus der Gruppe bestehend aus Hexan, Ethanol, Methanol, Chloroform, Dichlormethan, Ethylacetat, allein oder in Mischung.

6. Verfahren zum Erhalt eines Pflanzenextrakts nach irgendeinem der Ansprüche 1 bis 5, **dadurch gekennzeichnet, dass** das Verfahren einen der Extraktion vorgeschalteten Schritt der Dehydratisierung der Avocadobaum-Blätter umfasst.

7. Verfahren zum Erhalt eines Pflanzenextrakts nach Anspruch 6, **dadurch gekennzeichnet, dass** die Dehydratisierung ausgewählt ist aus der Gruppe bestehend aus Trocknen unter einem warmen Luftstrom oder unter geregelter Atmosphäre, Trocknen bei Atmosphärendruck oder unter Vakuum in dicker Schicht oder dünner Schicht, Trocknen durch Mikrowellen, Trocknen durch Zerstäuben, Lyophilisierung und osmotische Dehydratisierung in Lösung oder in fester Phase.

8. Verfahren zum Erhalt eines Pflanzenextrakts nach Anspruch 7, **dadurch gekennzeichnet, dass** die Dehydratisierung aus einem Trocknen in ventilierten Trocknungsvorrichtungen in dünner Schicht und unter einem warmen Luftstrom bei einer Temperatur zwischen 70 und 75°C einschließlich besteht.

9. Verfahren zum Erhalt eines Pflanzenextrakts nach irgendeinem der Ansprüche 1 bis 8, **dadurch gekennzeichnet, dass** das Verfahren gemäß der Abfolge der folgenden Schritte durchgeführt wird:
- Schritt 1: Wärmebehandlung der Avocadobaum-Blätter,
- Schritt 2: Flüssig-Fest-Extraktion der Avocadobaum-Blätter,
- Schritt 3: Verdampfen des Lösungsmittels unter Vakuum bis zum Erhalt eines Pflanzenextrakts ohne Lösungsmittel.

10. Verfahren zum Erhalt eines Pflanzenextrakts nach irgendeinem der Ansprüche 1 bis 8, **dadurch gekennzeichnet, dass** das Verfahren gemäß der Abfolge der folgenden Schritte durchgeführt wird:
- Schritt 1: Flüssig-Fest-Extraktion der Avocadobaum-Blätter,
- Schritt 2: Verdampfen des Lösungsmittels unter Vakuum bis zum Erhalt eines Pflanzenextrakts ohne Lösungsmittel,
- Schritt 3: Wärmebehandlung des Pflanzenextrakts.

11. Verfahren zum Erhalt eines Pflanzenextrakts nach irgendeinem der Ansprüche 1 bis 8, **dadurch gekennzeichnet, dass** das Verfahren gemäß der Abfolge der folgenden Schritte durchgeführt wird:
- Schritt 1: Dehydratisierung von Avocadobaum-Blättern,
- Schritt 2: Flüssig-Fest-Extraktion der Avocadobaum-Blätter,
- Schritt 3: Verdampfen des Lösungsmittels unter Vakuum bis zum Erhalt eines Pflanzenextrakts,
- Schritt 4: Wärmebehandlung des Pflanzenextrakts.
